(19) **Europäisches Patentamt / European Patent Office / Office européen des brevets**

(11) **EP 3 516 395 B1**

(12) # FASCICULE DE BREVET EUROPEEN

(45) Date de publication et mention de la délivrance du brevet:
**14.10.2020 Bulletin 2020/42**

(21) Numéro de dépôt: **17771454.0**

(22) Date de dépôt: **21.09.2017**

(51) Int Cl.:
*G01N 33/566* [(2006.01)]   *C40B 30/04* [(2006.01)]

(86) Numéro de dépôt international:
**PCT/EP2017/073942**

(87) Numéro de publication internationale:
**WO 2018/055053 (29.03.2018 Gazette 2018/13)**

(54) **PROCÉDÉ DE DÉTERMINATION DE L'AFFINITÉ ENTRE DES LIGANDS ET UNE CIBLE**

VERFAHREN ZUR BESTIMMUNG DER AFFINITÄT ZWISCHEN LIGANDEN UND EINEM ZIEL

METHOD FOR DETERMINING AFFINITY BETWEEN LIGANDS AND A TARGET

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **23.09.2016 FR 1670545**

(43) Date de publication de la demande:
**31.07.2019 Bulletin 2019/31**

(73) Titulaire: **Plant Advanced Technologies Pat 54500 Vandoeuvre-les-Nancy (FR)**

(72) Inventeur: **SALWINSKI, Aleksander 54000 Nancy (FR)**

(74) Mandataire: **Brungard, Yves Francois Actalium 29, rue de Sarre 57070 Metz (FR)**

(56) Documents cités:
- LIU SHU ET AL: "Characterization of compounds and potential neuraminidase inhibitors from the n-butanol extract of Compound Indigowoad Root Granuleusing ultrafiltration and liquid chromatography-tandem mass spectrometry", JOURNAL OF PHARMACEUTICAL AND BIOMEDICAL ANALYSIS, NEW YORK, NY, US, vol. 59, 20 octobre 2011 (2011-10-20), pages 96-101, XP028599161, ISSN: 0731-7085, DOI: 10.1016/J.JPBA.2011.10.015 cité dans la demande

- MULABAGAL V ET AL: "Development of binding assays to screen ligands for Plasmodium falciparum thioredoxin and glutathione reductases by ultrafiltration and liquid chromatography/mass spectrometry", JOURNAL OF CHROMATOGRAPHY B: BIOMEDICAL SCIENCES & APPLICATIONS, ELSEVIER, AMSTERDAM, NL, vol. 878, no. 13-14, 15 avril 2010 (2010-04-15), pages 987-993, XP026991593, ISSN: 1570-0232 [extrait le 2010-03-06] cité dans la demande

- RANJITH MUNIGUNTI ET AL: "Screening of natural compounds for ligands toTrxR by ultrafiltration and LCMS based binding assay", JOURNAL OF PHARMACEUTICAL AND BIOMEDICAL ANALYSIS, NEW YORK, NY, US, vol. 55, no. 2, 26 janvier 2011 (2011-01-26), pages 265-271, XP028172804, ISSN: 0731-7085, DOI: 10.1016/J.JPBA.2011.01.033 [extrait le 2011-02-03] cité dans la demande

- XIAOLAN YANG ET AL: "Estimation of affinities of ligands in mixtures via magnetic recovery of target-ligand complexes and chromatographic analyses: chemometrics and an experimental model", BMC BIOTECHNOLOGY, BIOMED CENTRAL LTD. LONDON, GB, vol. 11, no. 1, 5 mai 2011 (2011-05-05), page 44, XP021097759, ISSN: 1472-6750, DOI: 10.1186/1472-6750-11-44

- HAN WEI ET AL: "Pharmaceutical applications of affinity-ultrafiltration mass spectrometry: Recent advances and future prospects", JOURNAL OF PHARMACEUTICAL AND BIOMEDICAL ANALYSIS, vol. 131, 20 septembre 2016 (2016-09-20), pages 444-453, XP055361141, US ISSN: 0731-7085, DOI: 10.1016/j.jpba.2016.09.021

• **DONGTING LIU ET AL: "Screening for Ligands of Human Retinoid X Receptor-[alpha] Using Ultrafiltration Mass Spectrometry", ANALYTICAL CHEMISTRY, vol. 79, no. 24, 1 décembre 2007 (2007-12-01), pages 9398-9402, XP055361158, US ISSN: 0003-2700, DOI: 10.1021/ac701701k**

**Description**

**DOMAINE DE L'INVENTION**

L'invention concerne la caractérisation de l'affinité de ligands avec une cible telle qu'une enzyme. Elle concerne en particulier un procédé permettant de quantifier cette affinité.

**ART ANTÉRIEUR**

Dans les domaines de la médecine, de la cosmétique ou de l'agrobiologie, il est nécessaire de pouvoir déterminer si un ligand a une affinité avec une cible, par exemple avec une enzyme. Lorsqu'on a déterminé que le ligand avait une affinité avec la cible, on peut alors l'utiliser dans une composition pour par exemple inhiber l'effet de l'enzyme. Que les ligands soient obtenus par synthèse ou par extraction de plantes, ils se présentent en grandes variétés et il est utile de pouvoir isoler rapidement ceux qui peuvent avoir une action intéressante.

Le document EP 1 944 610 A1 montre par exemple une méthode basée sur la technique de laboratoire appelée exposition sur phage, ou « phage display » en anglais, pour isoler une famille de ligands selon laquelle :

▷ on prépare une bibliothèque de ligands de nature polypeptidique fixés à la surface de bactériophages à l'aide de la technique d'exposition sur phage ;

▷ on prépare un mélange avec une molécule cible et un échantillon formé d'un mélange de ligands analogues de nature polypeptidique fixés à des bactériophages pour que les ligands s'accrochent à la cible;

▷ on sépare les bactériophages pour lesquels des ligands de nature polypeptidique fixés se sont accrochés à une cible;

▷ on provoque la séparation des cibles de l'ensemble ligands-bactériophage;

▷ on amplifie les bactériophages séparés de leur cible à l'étape précédente;

▷ on détermine les séquences peptidiques de ligands de nature polypeptidique fixés aux bactériophages amplifiés.

Cette méthode permet d'identifier tous les ligands de nature polypeptidique qui étaient présents dans la bibliothèque préparée au départ et qui présentent une affinité avec la molécule cible. Cependant, même s'il est possible de déterminer quels sont les ligands qui ont une forte affinité, il n'est pas possible de les classer en fonction de leur affinité afin de sélectionner les plus efficaces.

Dans le document US 2003/148269, on suggère de réaliser un classement par affinité entre les ligands candidats pour une macromolécule. Un mélange comportant des complexes des différents ligands candidats et de la macromolécule est centrifugé de telle sorte que les complexes sont répartis du centre au bord. La mise en œuvre de cette méthode est délicate, en particulier pour séparer physiquement les complexes après centrifugation. De plus la classification obtenue reste approximative, avec un classement indiquant des liaisons faibles, moyennes ou fortes. Elle ne peut s'appliquer qu'à de petites populations de ligands.

Dans l'article « Micro-Scale Frontal Affinity Chromatography with Mass Spectrometric Détection : A New Method for the Screening of Compound Libraries », David C. Schriemer, David R. Bundle, Liang Li, and Ole Hindsgaul dans Angewandte Chemie International Edition, 1998, 37, No.24, pages 3383-3387, il est proposé de faire passer un échantillon contenant des ligands dans une colonne sur la phase stationnaire de laquelle sont fixés des molécules d'une cible. A la sortie de la colonne, le mélange est analysé. Les ligands qui arrivent le plus tardivement sont ceux qui ont été fixés par la cible. Il est ainsi possible de classer les ligands par affinité avec la cible. Cependant, on constate que la fixation de la cible sur la phase stationnaire de la colonne dénature l'activité de la cible de sorte que les résultats sont au moins partiellement impactés.

L'article « Screening of DHFR-binding drugs by MALDI-TOFMS », Paul Hannewald & Benoît Maunit & Jean-François Muller dans Anal Bioanal Chem (2008) 392 :1335-1344, montre une méthode de test de la liaison de molécules avec une cible dihydrofolate réductase. Le procédé comprend les étapes suivantes :

▷ préparation d'un mélange avec la cible et un échantillon formé d'un mélange de molécules à caractériser;

▷ rinçage et filtration du mélange pour ne conserver que les complexes cible-molécules;

▷ une partie des complexes cible-molécules sont directement analysés en MALDI-TOF pour analyser les ligands faiblement liés qui vont se détacher naturellement de la cible lors de la réception du laser;

▷ dénaturation de la dihydrofolate réductase pour libérer les ligands de l'autre partie des complexes cible-molécules;

▷ analyse en MALDI-TOF des ligands libérés;

▷ évaluation d'un rapport représentatif de l'affinité de chaque molécule avec la cible.

Cette méthode permet d'identifier tous les ligands qui étaient présents au départ et qui présentent une affinité avec la molécule cible. Il est aussi possible de déterminer quels sont les ligands qui ont la plus forte affinité, afin de sélectionner les plus efficaces. Cependant cette méthode est complexe car elle nécessite de réaliser deux analyses en MALDI-TOF. De plus le MALDI-TOF est une technique nécessitant une matrice créant un bruit de fond rendant délicat l'identification des ligands et qui ne permet pas la discrimination d'isomères présentant la même masse.

Dans la publication de LIU SHU ET AL : « Characterization of compounds and potential neuraminidase inhibitors from the n-butanol extract of Compound Indigowoad Root Granuleusing ultrafiltration and liquid chromatography-tandem mass spectrometry », JOURNAL OF PHARMACEUTICAL AND BIOMEDICALANALYSIS, NEW YORK, NY, US,vol. 59, 20 octobre 2011, pages 96-101, on montre l'analyse de la cible neuraminidase avec un extrait de racines d'une plante. Les complexes cible-ligands sont séparés dans une étape de lavage avec du méthanol lors d'une centrifugation, suivi d'une étape d'évaporation du méthanol.

Dans la publication de MULABAGAL V ET AL : « Development of binding assays to screen ligands for Plasmodium falciparum thioredoxin and glutathione reductases by ultrafiltration and liquid chromatography/mass spectrometry », JOURNAL OF CHROMATOGRAPHY B : BIOMEDICAL SCIENCES & APPLICATIONS, ELSEVIER, AMSTERDAM, NL,vol. 878, no. 13-14, pages 987-993, on montre l'analyse des cibles thiorédoxine et glutathione. Les complexes cible-ligands sont séparés dans une étape de lavage avec du méthanol lors d'une centrifugation, suivi d'une étape d'évaporation du méthanol sous azote. Les affinités relatives des ligands sont caractérisées en fonction des rapports de pics mesurés lors de l'analyse du surnageant, mais cette caractérisation ne s'affranchit pas des conditions d'expérimentation.

Dans le document RANJITH MUNIGUNTI ET AL : "Screening of natural compounds for ligands to PfTrxR by ultrafiltration and LCMS based binding assay", JOURNAL OF PHARMACEUTICAL AND BIOMEDICALANALYSIS, NEW YORK, NY, US,vol. 55, no. 2, pages 265-271, un mélange de ligands est utilisé avec comme cible une protéine PfTrxR impliquée dans le métabolisme du parasite de la malaria. Les valeurs des pics sur les chromatogrammes d'analyse LC-MS pour chaque ligand ont été comparées à des valeurs de référence par ligand obtenues avec une cible dénaturée.

## OBJECTIFS DE L'INVENTION

Pour résoudre ces problèmes l'invention vise à fournir un procédé de détermination de l'affinité entre des ligands et une cible qui soit simple à mettre en œuvre, fiable et précis.

## DESCRIPTION DE L'INVENTION

Avec ces objectifs en vue, l'invention a pour objet un procédé de détermination de l'affinité entre des ligands et une cible selon lequel :

▷ on prépare un mélange avec une cible et un échantillon formé d'un mélange de ligands pour que les ligands ayant une affinité avec la cible s'y accrochent ;

▷ on filtre le mélange pour ne conserver que les complexes cible-ligands;

▷ on provoque la séparation des complexes cible-ligands,

▷ on sépare les ligands et les cibles, on récupère par filtration les ligands séparés de la cible dans une solution traitée et on analyse ladite solution traitée pour identifier la présence des ligands,

caractérisé en ce que le mélange de ligands initial comprend les ligands en proportions ne dépassant pas un rapport de 5 pour 1 entre eux, en ce qu'on ajoute à l'échantillon un ligand de référence dont l'affinité avec la cible est connue, et en ce que l'analyse de la solution traitée comprend la quantification relative des ligands, les ligands en quantité plus importante comportant la plus grande affinité avec la cible, les rapports de concentration molaire entre les ligands et la cible étant supérieurs à 1,56.

L'utilisation d'un échantillon de ligands en proportions contrôlées permet d'éliminer une variabilité dans les résultats du procédé. Les variations dans les quantités de ligands identifiés après le traitement ne résultent que de la différence d'affinité entre la cible et lesdits ligands. Par ailleurs, la quantification des ligands dans la solution traitée donne une valeur chiffrée à l'affinité, ce qui est beaucoup plus précis que la classification connue dans l'art antérieur. Elle permet en outre de comparer des ligands non présents dans l'échantillon, mais dans celui d'une expérience menée indépendamment dans les mêmes conditions.

Le nombre de variétés de ligands dans l'échantillon n'est pas limité et on peut traiter un grand nombre de ceux-ci dans une seule expérience. On peut ainsi obtenir en un jour une classification d'un grand nombre de ligands, sans avoir à conduire un essai d'activité de chaque ligand vis-à-vis de la cible. Seuls des essais avec les ligands identifiés comme les plus intéressants seront conduits. Le procédé peut s'appliquer également à des cibles qui ne présentent pas d'activité telles que des protéines n'ayant pas d'activité enzymatique, alors qu'un essai d'activité serait inopérant. Par ailleurs, la cible reste libre dans la solution, contrairement aux méthodes qui nécessitent sa fixation à un support solide. La cible n'est donc pas modifiée et les résultats ne sont pas influencés par ce biais.

De préférence, les rapports de concentration molaire entre les ligands et la cible sont supérieurs à 1,6, voire 1,9. On a démontré que dans ces conditions, les résultats étaient concordants, quelque soit le rapport de concentration au dessus de cette limite, alors qu'en dessous, les valeurs de rapport d'affinité étaient affectées. Ces conditions garantissent que les ligands sont en concurrence et que les résultats sont reproductibles.

De préférence, les ligands dans le mélange initial sont en proportions ne dépassant pas un rapport de 3 pour 1, et de préférence encore sont en proportions équimolaires.

L'échantillon est préparé par exemple avec un solvant choisi dans le groupe comprenant des glycols, le diméthylsulfoxide, l'éthanol, et leurs mélanges à l'eau. La concentration de chaque ligand est par exemple comprise entre 0,1 et 10 mM, préférentiellement entre 0,75 et 1 mM.

La cible est par exemple dissoute dans une solution d'ammonium acétate avec un pH adapté à la cible. Les conditions sont adaptées afin de favoriser l'activité de la cible, et peuvent donc évoluer en fonction de ladite cible.

Selon un perfectionnement, la quantification relative des ligands comprend en outre une étape d'analyse de l'échantillon par la même méthode d'analyse que celle de la solution traitée, la comparaison des ligands entre eux étant effectuée par les rapports de la quantité mesurée dans la solution traitée sur la quantité mesurée dans l'échantillon. Ainsi, on s'affranchit de la variation de la réponse du ligand à la méthode d'analyse. Le différentiel de réponse sur l'échantillon d'un ligand à l'autre est compensé sur les résultats sur la solution traitée.

Selon une disposition des préparatifs, on forme un échantillon de référence en diluant l'échantillon de 10 à 20 fois pour l'étape d'analyse de l'échantillon.

Selon un mode de réalisation, l'analyse des ligands de la solution traitée est réalisée par chromatographie en phase liquide ou en phase supercritique. On obtient en sortie un diagramme d'intensité sur un temps de rétention appelé chromatogramme. Cette analyse utilise des moyens très répandus en laboratoire et reste très abordable.

Selon cette technique, chaque ligand est identifié par un pic sur un chromatogramme, la quantité associée à chaque ligand étant choisie parmi l'aire du pic ou l'intensité maximale du pic.

Selon l'invention, on ajoute à l'échantillon un ligand de référence dont l'affinité avec la cible est connue. Ainsi, l'affinité des ligands de l'échantillon avec la cible peut être comparée à un niveau d'affinité connu.

De manière préférentielle, on calcule pour chaque ligand un rapport d'affinité $RA_{CEi}$ tel que :

$$RA_{CEi} = \frac{NF_{TB,CEi}}{NF_{eq,CEi}}$$

avec :

$$NF_{eq,CEi} = \frac{IS_{eq,CEi}}{IS_{eq,REF}}$$

et

$$NF_{TB,CEi} = \frac{IS_{TB,CEi}}{IS_{TB,REF}}$$

où :

NF est un facteur de normalisation ;

$IS_{eq,CEi}$ est l'intensité du signal analytique pour le ligand CEi dans l'échantillon ;

$IS_{eq,REF}$ est l'intensité du signal analytique pour le ligand de référence REF dans l'échantillon ;

$IS_{TB,CEi}$ est l'intensité du signal analytique pour le ligand CEi dans la solution traitée des ligands détachés TB;

$IS_{TB,REF}$ est l'intensité du signal analytique pour le ligand de référence REF dans la solution traitée des ligands détachés TB ;

le rapport d'affinité représentant une affinité avec la cible croissante avec la valeur du facteur d'affinité.

De manière particulière, les ligands de l'échantillon sont obtenus par extraction d'une plante auxquels on ajoute un ligand de référence connu pour son affinité avec la cible. On peut ainsi repérer rapidement quelles molécules produites par des plantes peuvent avoir une activité vis-à-vis de la cible.

De manière complémentaire, avant de séparer les complexes cible-ligands, on rince le mélange avec un solvant et on sépare les complexes du solvant par filtration et centrifugation. Cette étape permet d'éliminer les ligands qui n'auraient pas suffisamment d'affinité avec la cible. Pour le rinçage, on peut utiliser de l'eau purifiée ou un solvant dans lequel l'enzyme est stable, tel qu'une solution d'acétate d'ammonium.

De manière particulière, pour séparer les ligands et les cibles, on ajoute de l'acétonitrile au mélange. On provoque ainsi la coagulation et la précipitation des cibles et la libération des ligands qui s'y sont liés précédemment. Les agglomérats sont simples à éliminer par filtration et les ligands sont récupérés dans le surnageant.

Typiquement, la cible est une enzyme. Les ligands qui ont une affinité avec l'enzyme permettent en général d'inhiber l'action de celle-ci. Le procédé permet donc de sélectionner des ligands pour leur potentielle activité inhibitrice de l'enzyme cible. On s'intéressera aux ligands qui ont les plus grands facteurs d'affinité. D'autres cibles possibles sont des protéines solubles naturellement, c'est à dire les protéines non membranaires.

## BRÈVE DESCRIPTION DES FIGURES

Pour une meilleure compréhension de l'invention, il est fait référence à la description qui suit et aux dessins annexés, parmi lesquels :

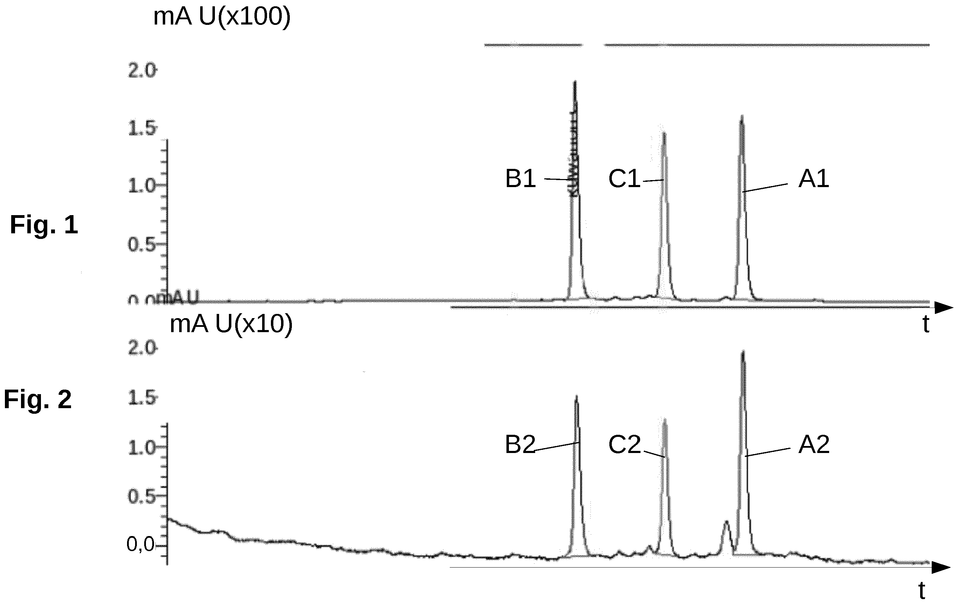

▷ la figure 1 est un chromatogramme correspondant à l'analyse d'un échantillon de ligands ;

▷ la figure 2 est un chromatogramme similaire à la figure 1 de l'échantillon après traitement selon le procédé de l'invention ;

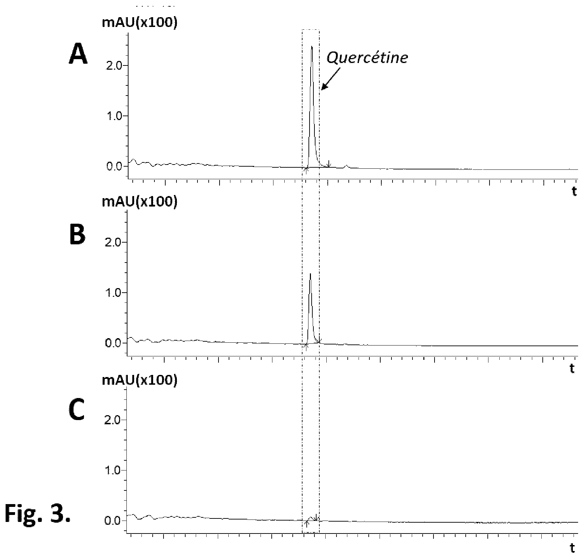

▷ la figure 3 regroupe trois chromatogrammes en lien avec l'exemple 5.

## DESCRIPTION DÉTAILLÉE

Un procédé de détermination de l'affinité entre des ligands et une cible repose sur le principe de la mise en contact en phase liquide des ligands et de la cible pour que les ligands qui ont une affinité avec la cible s'y lient, de l'élimination des ligands non fixés, de la séparation des complexes pour récupérer les ligands qui étaient fixés, puis de la quantification de ces derniers. Un échantillon de composés à tester est préparé avec des proportions contrôlées, y compris un ligand de référence dont l'affinité avec la cible existe et est connue.

Dans une première étape du procédé, l'échantillon et la cible sont mélangés. Les composés se font concurrence pour accéder aux sites de liaison de la cible. Les composés les plus forts déplacent les plus faibles et sont retenus de manière plus efficace par la cible.

Par la suite, on procède aux étapes suivantes :

i) séparation des complexes ligand-cible du mélange;

ii) élimination des composés non liés ;

iii) dénaturation des complexes ligand-cible pour relâcher les ligands;

iv) élimination de la cible dénaturée par précipitation et centrifugation ;

v) analyse du surnageant : comparaison des signaux analytiques des ligands du surnageant avec les signaux analytiques des ligands de l'échantillon.

**EXEMPLE 1 : TYROSINASE DE CHAMPIGNON**

1.1. Préparation de l'échantillon

Une solution de travail de tyrosinase de champignon à 1.25 mg/mL est préparée en dissolvant de la poudre de l'enzyme lyophilisée dans 50 mM d'acétate d'ammonium, avec un pH de 7.0. L'échantillon est préparé en utilisant l'un des solvants suivants : des glycols, DMSO (diméthylsulfoxide), l'éthanol, pur ou en solution dans l'eau. La concentration dans l'échantillon des ligands initiaux est équimolaire et doit être dans la gamme de 0,75 à 1 mM. Les ligands choisis étaient des inhibiteurs connus de la tyrosinase isolés depuis des racines de murier noir (*Morus nigra*) : la moracénine A, moracénine B et la kuwanone C. Un échantillon de référence est préparé également en diluant l'échantillon de 10 à 20 fois dans une solution d'eau et d'un quart de volume d'acétonitrile.

1.2. Analyse de l'échantillon

1. Étape de préconditionnement - Des puits à membrane filtrante sont pré-conditionnées avec un volume de 500 μl d'une solution à 85% d'acétate d'ammonium et 15% d'éthanol qui est passé à travers la membrane avec une centrifugation de 14000 g pendant 60 secondes.

2. Étape de liaison - La préparation de tyrosinase (1.25 mg/mL, 75 μL) est mélangée avec 5 μl de l'échantillon dans un tube d'Eppendorf en plastique, puis est incubée pendant 5 min. Dans ce mélange, on a 9,16 μM de tyrosinase et une gamme de 46,88 μM à 62,5 μM de ligands (moracénine A, moracénine B et kuwanone C) soit un ratio ligands/cible allant de 5,12 à 6,83.

3. Étape de lavage - le mélange de l'enzyme et de l'échantillon est déposé dans un puits préconditionné à l'étape de préconditionnement, puis centrifugé à 14000 g jusqu'à l'élimination de l'essentiel du solvant. Les complexes enzyme-ligands restent à la surface de la membrane du filtre puis sont à nouveau suspendu dans un volume de 100 μL de la même solution à 85% d'acétate d'ammonium et 15% d'éthanol et centrifugé à 14000 g jusqu'à élimination du solvant. Cette procédure est répétée trois fois et élimine tous les composés qui ne sont pas liés à l'enzyme.

4. Étape de séparation - Les complexes enzyme-ligands retenus dans le filtre sont à nouveau suspendus dans un volume de 10 μL d'eau ultra-pure, puis sont mélangés avec un volume de 40 μL d'acétonitrile pour dénaturer et précipiter l'enzyme et, en même temps libérer les ligands. Le mélange est centrifugé à 21000 g pendant 10 min pour séparer le précipité du surnageant.

5. Étape d'analyse - Ledit surnageant formant la solution traitée est analysé par chromatographie en phase liquide couplée à une spectrométrie de masse, connue également par l'acronyme UPLC-MS. L'échantillon de référence est analysé également de la même manière, en utilisant les mêmes conditions d'analyse. Les affinités relatives sont calculées selon la méthode décrite ci-après. De préférence, l'échantillon de référence et la solution traitée sont analysés séquentiellement pour éliminer des dispersions dues aux réglages de l'appareillage.

**EXEMPLE 2 : COLLAGÉNASE ISSUE DE *CLOSTRIDIUM HISTOLYTICUM***

2.1. Préparation de l'échantillon

Une solution de travail de collagénase issue de *Clostridium histolyticum* à 6.25 mg/mL est préparée en dissolvant de la poudre de l'enzyme lyophilisée dans 50 mM d'acétate d'ammonium, avec un pH de 7.5, ajusté par une solution aqueuse d'ammoniac. L'échantillon est préparé en utilisant l'un des solvants suivants : des glycols, le DMSO (diméthylsulfoxide), l'éthanol, pur ou en solution dans l'eau. La concentration dans l'échantillon des ligands initiaux est équimolaire et doit être dans la gamme de 0,75 à 1 mM. Les ligands choisis étaient des isomères d'acide 3,5-dicaféoylquinique (noté 3,5-DCQ par la suite) et des isomères d'esters d'acide 3,5-dicaféoylquinique et de dipropylène glycol (noté 3,5-DCQ-DPG par la suite). Un échantillon de référence est préparé également en diluant l'échantillon de 10 à 20 fois dans une solution d'eau et d'un quart de volume d'acétonitrile.

2.2. Analyse de l'échantillon

1. Étape de préconditionnement - Des puits à membrane filtrante sont pré-conditionnées avec un volume de 500 μl d'acétate d'ammonium 50mM pH 7,5 qui est passé à travers la membrane avec une centrifugation de 14000 g pendant 60 secondes.

2. Étape de liaison - Un volume de 75 μl de la solution de collagénase à une concentration de 6,25mg/ml dans 50 mM d'acétate d'ammonium, avec un pH de 7.5 est mélangé avec 10 μl de l'échantillon pendant 10 min dans un tube d'Eppendorf en plastique. Dans ce mélange, on a 55,79 μM de collagénase et une gamme de 93,75 μM à 125 μM de ligands (3,5-DCQ et 3,5-DCQ-DPG) soit un ratio ligands/cible entre 1,9 et 2,54.

3. Étape de lavage - Le mélange de l'enzyme et de l'échantillon est déposé dans un puits préconditionné à l'étape de préconditionnement, puis centrifugé à 14000 g jusqu'à l'élimination de l'essentiel du solvant. Les complexes enzyme-ligands restent à la surface de la membrane du filtre puis sont à nouveau suspendu dans un volume de 100 μL de la même solution à 50 mM d'acétate d'ammonium, avec un pH de 7.5 et centrifugé à 14000 g jusqu'à élimination du solvant. Cette procédure est répétée trois fois et élimine tous les composés qui ne sont pas liés à l'enzyme.

4. Étape de séparation - Les complexes enzyme-ligands retenus dans le filtre sont à nouveau suspendus dans un volume de 10 μL d'eau ultra-pure, puis sont mélangés avec un volume de 40 μL d'acétonitrile pour dénaturer et précipiter l'enzyme et, en même temps libérer les ligands. Le mélange est centrifugé à 21000 g pendant 10 min pour séparer le précipité du surnageant.

5. Étape d'analyse - Ledit surnageant formant la solution traitée est analysé par chromatographie en phase liquide couplée à une spectrométrie de masse. L'échantillon de référence est analysé également de la même manière, en utilisant les mêmes conditions d'analyse. Les affinités relatives sont calculées selon la méthode décrite ci-après. Le résultat est sur la première ligne du tableau 3.

## EXEMPLE 3 : COLLAGÉNASE ISSUE DE *CLOSTRIDIUM HISTOLYTICUM*

3.1. Préparation de l'échantillon

Plusieurs échantillons sont préparés comme dans l'exemple 2, mais avec une proportion entre les ligands 3,5-DCQ et 3,5-DCQ-DPG non plus équimolaire mais déséquilibrée, comme indiqué dans le tableau 3 ci-après.

3.2. Analyse de l'échantillon

L'analyse est conduite de la même manière que dans l'exemple 2. Les résultats sont de la deuxième à la quatrième ligne du tableau 3.

## EXEMPLE 4 : INHIBITION DE LA COLLAGÉNASE ISSUE DE *CLOSTRIDIUM HISTOLYTICUM*

L'activité inhibitrice de la collagénase issue de *Clostridium histolyticum* a été expérimentée en mettant en présence de la collagénase et du substrat synthétique FALGPA (N-(3-[2-Furyl]acryloyl)-Leu-Gly-Pro-Ala) préparé à une concentration de 1 mM, du tampon tris-HCl seul pour établir une référence, et en présence respectivement de tampon tris-HCl additionné de 3,5-DCQ et 3,5-DCQ-DPG en concentration à 5μM. La référence montrant une activité de la collagénase de 100%, on a mesuré une activité de la collagénase de 94% en présence de 3,5-DCQ et une activité de 50% en présence de 3,5-DCQ-DPG. A cette concentration, la collagénase de *Clostridium histolyticum* est inhibée de manière significative par le 3,5-DCQ-DPG, tandis qu'elle ne l'est que très peu par le 3,5-DCQ.

## EXEMPLE 5 : HYALURONIDASE ISSUE DE TESTICULES BOVINS

### 1.1. Préparation de l'échantillon

Une solution de travail de hyaluronidase de testicules bovins à 5 mg/mL est préparée en dissolvant de la poudre de l'enzyme lyophilisée dans un tampon phosphate salin à 50 mM, avec un pH de 7,0. Une solution de travail de trypsine (la cible témoin) est préparée de la même manière que celle de hyaluronidase. L'échantillon est préparé en utilisant l'un des solvants suivants : des glycols, DMSO (diméthylsulfoxide), l'éthanol pur ou en solution dans l'eau. La concentration dans l'échantillon de ligand initial doit être dans la gamme de 0,75 à 1 mM. Le ligand choisi (quercétine) est un inhibiteur connu de l'hyaluronidase (Asian J. Chem. 2013, 25, 10262-10266). Un échantillon de référence est préparé également en diluant l'échantillon de 10 à 20 fois dans une solution de trois quarts de volume d'acétonitrile et d'un quart de volume d'eau.

### 1.2. Analyse de l'échantillon

1. Étape de préconditionnement - Des puits à membrane filtrante sont pré-conditionnés avec un volume de 500 $\mu$l d'une solution du tampon de phosphate salin à 50 mM, avec un pH de 7,0 qui est passé à travers la membrane avec une centrifugation de 14000 g pendant 60 secondes.

2. Étape de liaison - La préparation de hyaluronidase (5 mg/mL, 90 $\mu$L) est mélangée avec 10 $\mu$l de l'échantillon dans un tube d'Eppendorf en plastique, puis est incubée pendant 10 min.

3. Étape de lavage - le mélange de l'enzyme et de l'échantillon est déposé dans un puits préconditionné à l'étape de préconditionnement, puis centrifugé à 14000 g jusqu'à l'élimination de l'essentiel du solvant. Les complexes enzyme-ligands restent à la surface de la membrane du filtre puis sont à nouveau suspendus dans un volume de 100 $\mu$L de la même solution du tampon de phosphate salin à 50 mM et centrifugé à 14000 g jusqu'à élimination du solvant. Cette procédure est répétée trois fois et élimine tous les composés qui ne sont pas liés à l'enzyme.

4. Étape de séparation - Les complexes enzyme-ligands retenus dans le filtre sont à nouveau suspendus dans un volume de 10 $\mu$L d'eau ultra-pure, puis sont mélangés avec un volume de 40 $\mu$L d'acétonitrile pour dénaturer et précipiter l'enzyme et, en même temps libérer les ligands. Le mélange est centrifugé à 21000 g pendant 10 min pour séparer le précipité du surnageant.

5. Étape d'analyse - Ledit surnageant formant la solution traitée est analysé par chromatographie en phase liquide couplée à une spectrométrie de masse, connue également par l'acronyme UPLC-MS. L'échantillon de référence est analysé également de la même manière, en utilisant les mêmes conditions d'analyse.

La même procédure - comprenant les étapes 1 à 5 - est menée pour la cible témoin.

## EXEMPLE 6 : COLLAGÉNASE ISSUE DE *CLOSTRIDIUM HISTOLYTICUM*

La préparation des échantillons (ligands et cible) et leur analyse ont été réalisés selon l'exemple 2. Les effets des variations du rapport de ligands/cible ont été évalués.

| Échantillon (M_x) | Condition de l'essai (C - en concurrence; N - sans concurrence) | Concentration ($\mu$M) | | Rapport Ligands/ collagénase |
|---|---|---|---|---|
| | | Collagénase | Ligands : DCQ/DCQ-DPG | |
| M_REF | C | 32,1 | 100/100 | 3,11 |
| M_1 | C | 32,1 | 75/75 | 2,33 |
| M_2 | C | 32,1 | 50/50 | 1,56 |
| M_3 | N | 32,1 | 15/15 | 0,47 |

**INTERPRÉTATION DES DONNÉES**

Une analyse par chromatographie en phase liquide fournit un chromatogramme tel que montré par les figures 1 et 2. Le chromatogramme représente une intensité de signal en fonction du temps de rétention dans une colonne. Le signal présente différents pics d'intensité variable. Chaque pic est associé à l'un des ligands présent dans l'échantillon et la solution traitée.

1. Normalisation de l'intensité du signal analytique pour les ligands dans l'échantillon de référence.
Pour chaque chaque ligand $CEi$ dans l'échantillon de référence, on établit des facteurs de normalisation $NF_{eq}$ dudit ligand $CEi$ par comparaison au ligand de référence $REF$, selon l'équation suivante :

$$NF_{eq,CEi} = \frac{IS_{eq,CEi}}{IS_{eq,REF}} \qquad (1)$$

où :

$NF$ est un facteur de normalisation ;
$IS_{eq,CEi}$ est l'intensité du signal analytique pour le ligand $CEi$ dans l'échantillon ;
$IS_{eq,REF}$ est l'intensité du signal analytique pour le ligand de référence $REF$ dans l'échantillon.

L'intensité peut être choisie parmi l'aire du pic ou la valeur maximale du pic. Le facteur de normalisation du ligand de référence vaut toujours 1.
2. Normalisation de l'intensité du signal analytique pour les ligands dans la solution traitée.
Pour chaque ligand $CEi$ dans la solution traitée, on établit des facteurs de normalisation $NF_{TB}$ dudit ligand $CEi$ par comparaison au ligand de référence $REF$, selon l'équation suivante :

$$NF_{TB,CEi} = \frac{IS_{TB,CEi}}{IS_{TB,REF}} \qquad (2)$$

où :

$IS_{TB,CEi}$ est l'intensité du signal analytique pour le ligand $CEi$ dans la solution traitée ; et
$IS_{TB,REF}$ est l'intensité du signal analytique pour le ligand de référence $REF$ dans la solution traitée.

Le facteur de normalisation du ligand de référence vaut toujours 1.
3. Affinités relatives des ligands en rapport avec le ligand de référence.

Ensuite, on calcule pour chaque ligand un rapport d'affinité $RA_{CEi}$ tel que :

$$RA_{CEi} = \frac{NF_{TB,CEi}}{NF_{eq,CEi}} \qquad (3)$$

Le rapport d'affinité représente une affinité du ligand avec la cible, cette affinité étant croissante avec la valeur du facteur d'affinité. Les ligands ayant les rapports d'affinité les plus élevés seront les candidats privilégiés pour une inhibition de l'activité de la cible.
L'invention n'est pas limitée aux exemples donnés. On peut par exemple ajouter à la cible des ligands qui se fixent sur certains sites de manière à identifier des ligands qui seraient adaptés aux sites restants libres.

## RÉSULTATS

Dans l'exemple 1 de la tyrosinase, le ligand moracénine B a été considéré comme le ligand de référence. Les figures 1 et 2 montrent les chromatogrammes en ultraviolet, à une longueur d'onde de 270 nm, respectivement sur l'échantillon de référence et sur la solution traitée. Le chromatogramme de l'échantillon de référence sur la figure 1 comporte trois pics B1, C1, A1 correspondant respectivement à la moracénie B, à la kuwanone C et à la moracénine A. Le chromatogramme de la solution traitée sur la figure 2 comporte trois pics B2, C2, A2 correspondant respectivement à la moracénie B, à la kuwanone C et à la moracénine A.

La surface des pics issue de ces diagrammes de tous les ligands est utilisée pour calculer les facteurs de normalisation selon l'équation 1 et l'équation 2. Sur la base de ces valeurs, les facteurs d'affinité sont calculés selon l'équation 3 pour tous les ligands compris dans l'échantillon de référence. Le tableau 2 montre la synthèse des résultats et comprend en dernière colonne la comparaison avec des valeur expérimentales issues de la littérature indiquant l'indice $IC_{50}$ de réduction d'activité de l'enzyme de 50% en présence du ligand.

| Ligand | Facteur de normalisation, échantillon de référence | | Facteur de normalisation, solution traitée | | Affinités relatives | | $IC_{50}$ |
|---|---|---|---|---|---|---|---|
| moracénine B | $NF_{eq,REF}$ | 1,00 | $NF_{TB,REF}$ | 1,00 | $RA_{REF}$ | 1,00 | 200 à 300 µM |
| kuwanone C | $NF_{eq,kuwC}$ | 0,73 | $NF_{TB,kuwC}$ | 0,78 | $RA_{kuwC}$ | 1,07 | 50 à 100 µM [1] |
| moracénine A | $NF_{eq,morA}$ | 0,86 | $NF_{TB,morA}$ | 1,29 | $RA_{morA}$ | 1,50 | 10,3 µM [2] |

[1] J. Agric. Food Chem. 2010, 58, 5368-5373

[2] Molecules 2015, 20, 8730-8741

On constate que les résultats du procédé selon l'invention sont corrélés aux résultats connus de l'activité des ligands testés.

Dans le tableau 3, on évalue la sensibilité de la méthode à l'écart de concentrations des ligands par rapport à l'équimolarité. La première ligne du tableau 3 concerne le résultat avec des concentrations entre 3,5-DCQ et 3,5-DCQ-DPG égales, donc avec un mélange équimolaire, et correspond aux résultats de l'exemple 2. On montre que l'ester 3,5-DCQ-DPG présente l'affinité la plus grande pour la cible par rapport au 3,5-DCQ de référence. Ceci est cohérent avec la littérature qui indique que l'indice $IC_{50}$ de 3,5-DCQ vis-à-vis de la collagénase issue de *Clostridium histolyticum* est de 23.6 µM (J. Nat. Prod. 2005, 68, 794-796) alors que celui de l'ester 3,5-DCQ-DPG est d'environ 5 µM, comme l'a montré l'exemple 4. Le site actif de la collagénase est bloqué par l'ester à une plus faible concentration que pour le 3,5-DCQ.

Les résultats de l'exemple 3 montrent par ailleurs que, même si l'affinité relative du 3,5-DCQ-DPG évolue avec le déséquilibre de concentration, cette évolution reste dans la même orientation, en indiquant à chaque fois que le 3,5-DCQ-DPG est plus efficace pour inhiber la collagénase que le 3,5-DCQ. Toutefois, l'exemple 3.2 montre que la sensibilité de l'essai diminue sérieusement avec un rapport molaire de 5 :1. On considère ainsi que les objectifs de l'invention ne seraient pas atteints avec un écart plus important.

Tableau 3

| Rapport molaire 3,5-DCQ-DPG/ 3,5-DCQ (réf) | N° ex. | Concentration dans l'échantillon (µM) | | Facteur de normalisation, échantillon de référence | | Facteur de normalisation, solution traitée | | Affinités relatives | |
|---|---|---|---|---|---|---|---|---|---|
| | | 3,5-DCQ-DPG | 3,5-DCQ (réf) | 3,5-DCQ-DPG | 3,5-DCQ (réf) | 3,5-DCQ-DPG | 3,5-DCQ (réf) | 3,5-DCQ-DPG | 3,5-DCQ (réf) |
| 1/1 (équimolaire) | 2 | 58,8 | 58.8 | 1,16 | 1,00 | 6,03 | 1,00 | 5,21 | 1,00 |
| 0,5/1 | 3.1 | 29,4 | 58,8 | 0,62 | 1,00 | 2,49 | 1,00 | 4,05 | 1,00 |

(suite)

| Rapport molaire 3,5-DCQ-DPG/ 3,5-DCQ (réf) | N° ex. | Concentration dans l'échantillon (μM) | | Facteur de normalisation, échantillon de référence | | Facteur de normalisation, solution traitée | | Affinités relatives | |
|---|---|---|---|---|---|---|---|---|---|
| | | 3,5-DCQ-DPG | 3,5-DCQ (réf) | 3,5-DCQ-DPG | 3,5-DCQ (réf) | 3,5-DCQ-DPG | 3,5-DCQ (réf) | 3,5-DCQ-DPG | 3,5-DCQ (réf) |
| 0,2/1 | 3.2 | 11,8 | 58,8 | 0,27 | 1,00 | 0,81 | 1,00 | 3,01 | 1,00 |
| 3/1 | 3.3 | 58,8 | 19,6 | 3,33 | 1,00 | 13,64 | 1,00 | 4,09 | 1,00 |

Les résultats de l'exemple 5 sont présentés sur la figure 3. Le chromatogramme A correspond à l'analyse d'un échantillon de quercétine et montre un pic caractéristique. Le chromatogramme B est similaire au chromatogramme A pour un échantillon après traitement selon le procédé de l'invention en utilisant une hyaluronidase. Le chromatogramme C concerne un échantillon après traitement selon le procédé de l'invention en utilisant comme cible témoin de la trypsine. Les résultats montrent que la quercétine - l'inhibiteur de l'hyaluronidase - présente une affinité importante pour cette enzyme (chromatogramme B), et une très faible affinité pour la cible témoin (trypsine) (chromatogramme C). Ceci est cohérent avec la littérature qui indique que l'inhibition de l'hyaluronidase par la quercétine est de 54% à 200 μM (Asian J. Chem. 2013, 25, 10262-10266), alors que l'inhibition de la trypsine par cette molécule est de 46% à 2660 μM (International Journal of Food Science & Technology 2014, 49, 1063-1069).

Les résultats de l'exemple 6 sont présentés dans le tableau 4. Ils montrent que pour les rapports de concentration ligands/enzyme supérieurs à 1,6, on obtient des conditions de concurrence. Pour les conditions de concurrence, on observe que les paramètres RA (affinité relative) du DCQ-DPG vis-à-vis DCQ avec la collagénase restent relativement constants, même si on augmente les concentrations des ligands, comme le montre les résultats du tableau 4 (l'essai M_1 donne pratiquement le même rapport d'affinité que la référence). En d'autres termes, au-delà de ce seuil, l'essai n'est pas sensible à l'excès de ligands par rapport à la cible.

Tableau 4

| Échantillon (M_x) | $\dfrac{RA_{DCQ-DPG}^{Mx}}{RA_{DCQ-DPG}^{Mref}}$ |
|---|---|
| M_1 | 0,96 |
| M_2 | 0,71 |
| M_3 | 0,47 |

Avec un rapport ligands/cible inférieur à 1,56, les affinités relatives chutent avec la diminution de la concentration des ligands par rapport à une concentration fixe de cible (tableau 4). Cela est expliqué par des conditions de non concurrence, c'est-à-dire, un excès de la cible par rapport aux ligands.

**Revendications**

1. Procédé de détermination de l'affinité entre des ligands et une cible selon lequel

   ▷ on prépare un mélange avec une cible et un échantillon formé d'un mélange de ligands pour que les ligands ayant une affinité avec la cible s'y accrochent ;
   ▷ on filtre le mélange pour ne conserver que les complexes cible-ligands ;
   ▷ on provoque la séparation des complexes cible-ligands,
   ▷ on sépare les ligands et les cibles, on récupère par filtration les ligands dans une solution traitée et on analyse ladite solution traitée pour identifier la présence des ligands,

   **caractérisé en ce que** le mélange de ligands initial comprend les ligands en proportions ne dépassant pas un rapport de 5 pour 1 entre eux, **en ce qu'**on ajoute à l'échantillon un ligand de référence dont l'affinité avec la cible est connue, et **en ce que** l'analyse de la solution traitée comprend la quantification relative des ligands, les ligands en quantité plus importante comportant la plus grande affinité avec la cible, les rapports de concentration molaire entre les ligands et la cible étant supérieurs à 1,56.

2. Procédé selon la revendication 1, selon lequel les rapports de concentration molaire entre les ligands et la cible sont supérieurs à 1,6, voire 1,9.

3. Procédé selon la revendication 1 ou 2, selon lequel les ligands dans le mélange initial sont en proportions ne dépassant pas un rapport de 3 pour 1, de préférence sont en proportions équimolaires.

4. Procédé selon l'une des revendications 1 à 2, selon lequel la quantification relative des ligands comprend en outre une étape d'analyse de l'échantillon par la même méthode d'analyse que celle de la solution traitée, la comparaison des ligands entre eux étant effectuée par les rapports de la quantité mesurée dans la solution traitée sur la quantité mesurée dans l'échantillon.

5. Procédé selon la revendication 4, selon lequel on forme un échantillon de référence en diluant l'échantillon de 10 à 20 fois pour l'étape d'analyse de l'échantillon.

6. Procédé selon la revendication 4, selon lequel chaque ligand est identifié par un pic sur un chromatogramme, la quantité associée à chaque ligand étant choisie parmi l'aire du pic ou l'intensité maximale du pic.

7. Procédé selon la revendication 6, selon lequel on calcule pour chaque ligand un rapport d'affinité $RA_{CEi}$ tel que :

$$RA_{CEi} = \frac{NF_{TB,CEi}}{NF_{eq,CEi}}$$

avec :

$$NF_{eq,CEi} = \frac{IS_{eq,CEi}}{IS_{eq,REF}}$$

et

$$NF_{TB,CEi} = \frac{IS_{TB,CEi}}{IS_{TB,REF}}$$

ou :

$NF$ est un facteur de normalisation ;
$IS_{eq,CEi}$ est l'intensité du signal analytique pour le ligand $CEi$ dans l'échantillon ;
$IS_{eq,REF}$ est l'intensité du signal analytique pour le ligand de référence $REF$ dans l'échantillon ;
$IS_{TB,CEi}$ est l'intensité du signal analytique pour le ligand $CEi$ dans la solution traitée ;
$IS_{TB,REF}$ est l'intensité du signal analytique pour le ligand de référence $REF$ dans la solution traitée;
le rapport d'affinité représentant une affinité avec la cible croissante avec la valeur du facteur d'affinité.

8. Procédé selon l'une des revendications 4 à 7, selon lequel la chromatographie est couplée à la spectrométrie de masse.

9. Procédé selon l'une des revendications précédentes, dans lequel les ligands de l'échantillon sont obtenus par extraction d'une plante.

10. Procédé selon l'une des revendications précédentes, selon lequel avant de séparer les complexes cible-ligands, on rince le mélange avec un solvant et on sépare les complexes du solvant par filtration et centrifugation.

11. Procédé selon l'une des revendications précédentes, selon lequel pour séparer les ligands et les cibles, on ajoute de l'acétonitrile au mélange.

12. Procédé selon l'une des revendications précédentes, selon lequel la cible est une enzyme.


**Patentansprüche**

1. Ein Verfahren zur Bestimmung der Affinität zwischen Liganden und einer Zielverbindung, demzufolge

   ➤ eine Mischung aus einer Zielverbindung und einer aus einem Gemisch aus Liganden mit Affinität zur Zielverbindung bereiteten Probe bereitet wird, sodass Letztere an Erstere koppeln;

   ➤ diese Mischung dergestalt filtriert wird, dass lediglich die aus Zielverbindung und Liganden gebildeten Komplexe bewahrt werden;

   ➤ die aus Zielverbindung und Liganden gebildeten Komplexe abgetrennt werden,

   ➤ Liganden und Zielverbindung voneinander getrennt werden, die Liganden aus einer aufbereiteten Lösung durch Filtration zurückgewonnen werden und selbige aufbereitete Lösung auf die Gegenwart von Liganden hin analysiert wird,

   das **dadurch gekennzeichnet ist, dass** das ursprüngliche Ligandengemisch Liganden in Mischungsverhältnissen von untereinander maximal 5 zu 1 enthält, dass zur Probe ein Bezugsligand bekannter Affinität zur Zielverbindung hinzugegeben wird, und dass die Analyse der aufbereiteten Lösung die quantitative Bestimmung der Anteile der Liganden beinhaltet, wobei die in größerer Menge vorliegenden Liganden die größte Affinität zur Zielverbindung aufweisen und die Verhältnisse der Stoffmengenkonzentrationen zwischen den Liganden und der Zielverbindung mehr als 1,56 betragen.

2. Ein Verfahren gemäß Patentanspruch 1, demzufolge die Verhältnisse der Stoffmengenkonzentrationen zwischen den Liganden und der Zielverbindung mehr als 1,6, teils über 1,9, betragen.

3. Ein Verfahren gemäß Patentanspruch 1 oder 2, demzufolge die Liganden im ursprünglichen Gemisch in Verhältnissen von maximal 3 zu 1 und vorzugsweise in äquimolarem Verhältnis vorliegen.

4. Ein Verfahren gemäß einem der Patentansprüche 1 oder 2, demzufolge die quantitative Bestimmung der Anteile der Liganden darüber hinaus einen Probenanalyseschritt nach demselben Analyseverfahren beinhaltet, das auch auf die aufbereitete Lösung angewandt wird, wobei die Liganden untereinander anhand des jeweiligen Verhältnisses zwischen der in der aufbereiteten Lösung und der in der Probe ermittelten Menge verglichen werden.

5. Ein Verfahren gemäß Patentanspruch 4, demzufolge eine Bezugsprobe erstellt wird, indem für den Analyseschritt die Probe 10- bis 20-fach verdünnt wird.

6. Ein Verfahren gemäß Patentanspruch 4, demzufolge jeder Ligand durch einen Peak in einem Chromatogramm gekennzeichnet ist, wobei die jedem Liganden entsprechende Menge anhand des Flächenintegrals unter dem Peak oder der maximalen Stärke des Peaks ermittelt wird.

7. Ein Verfahren gemäß Patentanspruch 6, demzufolge zu jedem Liganden ein Affinitätsverhältnis *RACEi* ermittelt wird gemäß:

$$RA_{CEi} = \frac{N\,F_{TB,CEi}}{N\,F_{eq,CEi}}$$

mit:

$$N\,F_{eq,CEi} = \frac{I\,S_{eq,CEi}}{I\,S_{eq,REF}}$$

und

$$N\,F_{TB,CEi} = \frac{I\,S_{TB,CEi}}{I\,S_{TB,REF}}$$

wobei:

$NF$ einen Normierungsfaktor darstellt;
$IS_{eq,CEi}$ die Stärke des Analysesignals bzgl. des Liganden *CEi* in der Probe darstellt;
$ISeq,REF$ die Stärke des Analysesignals bzgl. des Bezugsliganden *REF* in der Probe darstellt;
$ISTB,CEi$ die Stärke des Analysesignals bzgl. des Liganden *CEi* in der aufbereiteten Lösung darstellt;
$ISTB, REF$ die Stärke des Analysesignals bzgl. des Bezugsliganden REF in der aufbereiteten Lösung darstellt;
wobei das Affinitätsverhältnis eine mit dem Wert des Affinitätsfaktors zunehmende Affinität zur Zielverbindung darstellt.

8. Ein Verfahren gemäß einem der Patentansprüche 4 bis 7, demzufolge die Chromatographie an ein massenspektrometrisches Messverfahren gekoppelt ist.

9. Ein Verfahren gemäß einem der vorgenannten Patentansprüche, in welchem die Liganden der Probe auf dem Wege der Extraktion aus einer Pflanze gewonnen werden.

10. Ein Verfahren gemäß einem der vorgenannten Patentansprüche, demzufolge vor der Abtrennung der von Zielverbindung und Liganden gebildeten Komplexe das Gemisch mit einem Lösemittel gewaschen wird und die Komplexverbindungen durch Filtrieren oder Zentrifugieren vom Lösemittel abgetrennt werden.

11. Ein Verfahren gemäß einem der vorgenannten Patentansprüche, demzufolge zur Abtrennung der Liganden von den Zielverbindungen dem Gemisch Acetonitril zugegeben wird.

12. Ein Verfahren gemäß einem der vorgenannten Patentansprüche, demzufolge es sich bei der Zielverbindung um ein Enzym handelt.

**Claims**

1. Process for determining the affinity between ligands and a target whereby:

   ➤ a mixture is prepared with a target and a sample formed from a mixture of ligands so that the ligands that have an affinity for the target bind to it,

   ➤ the mixture is filtered to only keep the target-ligand complexes,

   ➤ the target-ligand complexes are made to separate,

   ➤ the ligands are separated from the targets, and the ligands are retrieved by filtering in a treated solution and said treated solution is analysed to determine the presence of the ligands,

   **characterised by** the fact the initial mixture of ligands includes ligands in proportions not exceeding a ratio of 5 to 1 between them, by the fact a reference ligand whose affinity for the target is known is added to the sample, by the fact the analysis of the treated solution includes the relative quantification of the ligands, the ligands in a greater quantity having the greatest affinity for the target, the ratios of molar concentration between the ligands and the target being higher than 1.56.

2. Process as per claim 1 whereby the molar concentration ratios between the ligands and the target are higher than 1.6, or even 1.9.

3.  Process as per claim 1 or 2 whereby the ligands in the initial mixture are in proportions not exceeding a ratio of 3 to 1, preferably in equimolar proportions.

4.  Process as per one of the claims 1 to 2 whereby the relative quantification of the ligands also includes a sample analysis step using the same analysis method as for the treated solution, the comparison between the ligands being made by the ratios of the quantity measured in the treated solution to the quantity measured in the sample.

5.  Process as per claim 4 whereby a reference sample is formed by diluting the sample 10 to 20 times for the sample analysis step.

6.  Process as per claim 4 whereby each ligand is identified by a peak on a chromatogram, the quantity associated with each ligand being chosen between the peak area and the peak's maximum intensity.

7.  Process as per claim 6 whereby an affinity ratio $RA_{CEi}$ is calculated for each ligand such that:

$$RA_{CEi} = \frac{NF_{TB,CEi}}{NF_{eq,CEi}}$$

with:

$$NF_{eq,CEi} = \frac{IS_{eq,CEi}}{IS_{eq,REF}}$$

and

$$NF_{TB,CEi} = \frac{IS_{TB,CEi}}{IS_{TB,REF}}$$

where:

$NF$ is a normalisation factor;
$IS_{eq,CEi}$ is the intensity of the analytical signal for the ligand $CEi$ in the sample;
$IS_{eq,REF}$ is the intensity of the analytical signal for the reference ligand $REF$ in the sample;
$IS_{TB,CEi}$ is the intensity of the analytical signal for the ligand $CEi$ in the treated solution;
$IS_{TB,REF}$ is the intensity of the analytical signal for the reference ligand $REF$ in the treated solution;
the affinity ratio representing an affinity for the target increasing with the value of the affinity factor.

8.  Process as per one of the claims 4 to 7, whereby the chromatography is coupled with mass spectrometry.

9.  Process as per one of the above claims whereby the ligands of the sample are obtained by extraction from a plant.

10. Process as per one of the above claims whereby, before separating the target-ligand complexes, the mixture is rinsed with a solvent and the complexes are separated from the solvent by filtration and centrifugation.

11. Process as per one of the above claims whereby acetonitrile is added to the mixture to separate the ligands from the targets.

12. Process as per one of the above claims whereby the target is an enzyme.

**Fig. 1**

**Fig. 2**

EP 3 516 395 B1

Fig. 3.

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 1944610 A1
- US 2003148269 A

**Littérature non-brevet citée dans la description**

- **DAVID C. SCHRIEMER ; DAVID R. BUNDLE ; LIANG LI ; OLE HINDSGAUL.** Micro-Scale Frontal Affinity Chromatography with Mass Spectrometric Détection : A New Method for the Screening of Compound Libraries. *Angewandte Chemie International Edition,* 1998, vol. 37 (24), 3383-3387
- **PAUL HANNEWALD ; BENOÎT MAUNIT ; JEAN-FRANÇOIS MULLER.** Screening of DHFR-binding drugs by MALDI-TOFMS. *Anal Bioanal Chem,* 2008, vol. 392, 1335-1344
- **LIU SHU et al.** Characterization of compounds and potential neuraminidase inhibitors from the n-butanol extract of Compound Indigowoad Root Granuleusing ultrafiltration and liquid chromatography-tandem mass spectrometry. *JOURNAL OF PHARMACEUTICAL AND BIOMEDICALANALYSIS,* 20 Octobre 2011, vol. 59, 96-101
- Development of binding assays to screen ligands for Plasmodium falciparum thioredoxin and glutathione reductases by ultrafiltration and liquid chromatography/mass spectrometry. **MULABAGAL V et al.** JOURNAL OF CHROMATOGRAPHY B : BIOMEDICAL SCIENCES & APPLICATIONS. ELSEVIER, vol. 878, 987-993
- **RANJITH MUNIGUNTI et al.** Screening of natural compounds for ligands to PfTrxR by ultrafiltration and LCMS based binding assay. *JOURNAL OF PHARMACEUTICAL AND BIOMEDICALANALYSIS,* vol. 55 (2), 265-271
- *Asian J. Chem.,* 2013, vol. 25, 10262-10266
- *J. Agric. Food Chem.,* 2010, vol. 58, 5368-5373
- *Molecules,* 2015, vol. 20, 8730-8741
- *J. Nat. Prod.,* 2005, vol. 68, 794-796
- *International Journal of Food Science & Technology,* 2014, vol. 49, 1063-1069